(19) Europäisches Patentamt
European Patent Office
Office européen des brevets

(11) Publication number: 0 330 253
A1

(12) **EUROPEAN PATENT APPLICATION**

(21) Application number: 89200298.1

(51) Int. Cl.4: C07J 43/00 , A61K 31/58

(22) Date of filing: 09.02.89

(30) Priority: 26.02.88 GB 8804579

(43) Date of publication of application:
30.08.89 Bulletin 89/35

(84) Designated Contracting States:
AT BE CH DE ES FR GB GR IT LI NL SE

(71) Applicant: AKZO N.V.
Velperweg 76
NL-6824 BM Arnhem(NL)

(72) Inventor: Sleigh, Thomas
3 Coulter Avenue
Lanarkshire Wishaw Scotland ML2 8SZ(GB)

Inventor: Savage, David Samuel
37 Beech Avenue
Glasgow Newton Mearns Scotland G77
5PP(GB)

Inventor: Clark, John Kellock
27 Sycamore Drive
Lanarkshire Hamilton Scotland ML3 7HF(GB)

(74) Representative: Hermans, Franciscus G.M. et al
Patent Department AKZO N.V. Pharma
Division P.O. Box 20
NL-5340 BH Oss(NL)

(54) **Novel 16-homo-piperidino-androstane derivatives and processes for their preparation.**

(57) The invention relates to compounds having the formula:

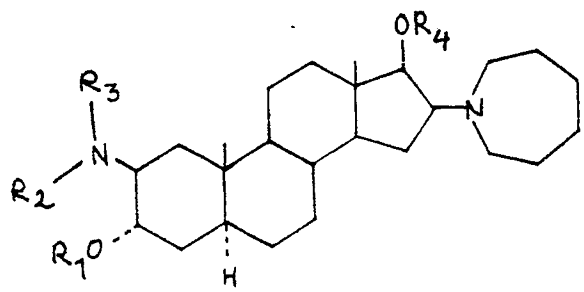

wherein $R_1$ is H or an acyl group having 1-12 carbon atoms,
$R_2$ and $R_3$ are an alkyl or aralkyl group having 1-12 carbon atoms, or when taken together form with the nitrogen atom a heterocyclic amino radical; and $R_4$ is H or an acyl group having 1-12 carbon atoms; and mono- or bisquaternary ammonium compounds thereof and acid addition salts of the non- or mono-quaternary ammonium compounds and to a process for preparing these compounds. The compounds are neuromuscular blocking agents with a fast onset and a short duration and which exhibit no or hardly any blood pressure drop.

EP 0 330 253 A1

## Novel 16-homo-piperidino-androstane derivatives and processes for their preparation.

The present invention is concerned with novel 16-homo-piperidino-androstane derivatives and with processes for their preparation.

16-piperidino-androstane derivatives have been disclosed in British Patent 1,138,605. These derivatives, which are neuromuscular blocking agents, exhibit a relatively slow onset and long duration and often show cardiovascular side-effects, like e.g. a considerable blood pressure drop.

Surprisingly a novel class of compounds with a homo-piperidino substituent at position 16 of the steroid skeleton has been found with an unexpectedly fast onset and short duration and which exhibit no or hardly any blood pressure drop.

Accordingly, the present invention is concerned with compounds having the formula:

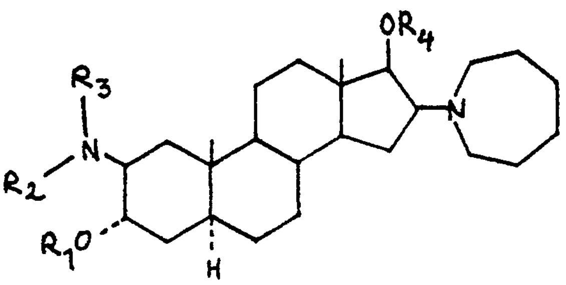

wherein

$R_1$ is H or an acyl group having 1-12 carbon atoms;

$R_2$ and $R_3$ are an alkyl or aralkyl group having 1-12 carbon atoms, or when taken together form with the nitrogen atom a heterocyclic amino radical; and

$R_4$ is H or an acyl group having 1-12 carbon atoms;

and mono- or bisquaternary ammonium compounds thereof and acid addition salts of the non- or mono-quaternary ammonium compounds.

$R_1$ in the above formula may be H or an acyl group having 1-12 carbon atoms. Suitable acyl groups are formyl, acetyl, propionyl, butyryl, isobutyryl, valeryl, pivaloyl, cyclopentane carbonyl, cyclohexanecarbonyl, trimethylacetyl, phenylpropionyl, propenoyl, 3-butenoyl and 4-pentenoyl. If $R_1$ is an acyl group preference is given to saturated acyl groups having 1-6 carbon atoms. The most preferred group for $R_1$ is H, acetyl, propionyl or butyryl.

$R_2$ and $R_3$ may be an alkyl or aralkyl group having 1-12 carbon atoms, like methyl, ethyl, propyl, hexyl, phenyl, cumenyl, tolyl and xylyl. preferably $R_2$ and $R_3$ when taken together with the nitrogen atom form a heterocyclic amino radical, like those derived from pyrrole, pyridine, indole, pyrrolidine, piperidine, homopiperidine and morpholine. The most preferred radicals are the saturated radicals mentioned and especially those derived from piperidine.

$R_4$ in the above formula may be H or an acyl group having 1-12 carbon atoms. Suitable acyl groups are those acyl groups mentioned above. Preferably $R_4$ is H or a saturated acyl group having 1-6 carbon atoms.

The quaternary ammonium salts of the compounds according to the above formula also form part of the compounds according to the present invention, as do the acid addition salts of the non- and mono-quaternary ammonium compounds. The quaternary ammonium compounds may be 2β-quaternary-, 16β-quaternary- or 2β,16β-bisquaternary ammonium compounds.

Preference is given to the 16β-mono-quaternary ammonium compounds.

The quaternising group in the quaternary ammonium derivatives is an aliphatic hydrocarbon group having 1-6 carbon atoms, such as methyl, ethyl, ethynyl, propyl, allyl, propargyl, butyl, isobutyl, pentyl, cyclopropyl, cyclopropylmethyl and cyclohexyl. The anion in the quaternary ammonium derivatives may in principle be any pharmaceutically acceptable organic or inorganic anion, such as methylsulphonate, p-toluene sulphonate, $Cl^-$, $Br^-$ or $I^-$, and is preferably $Br^-$.

The non-quaternised and mono-quaternised compounds may be converted into the acid addition salt thereof, derived from any pharmaceutically acceptable organic or inorganic acid, such as hydrochloric acid, hydrobromic acid and hydro-iodic acid, nitric acid, sulphuric acid, phosphoric acid, malonic acid, succinic acid, glutaric acid, maleic acid, fumaric acid, tartaric acid, malic acid, pyruvic acid, lactic acid and citric acid.

The compounds according to the invention can be prepared by methods obvious to those skilled in the art.

The process for the preparation of the compounds according to claim 1 is characterized in that a compound having the formula:

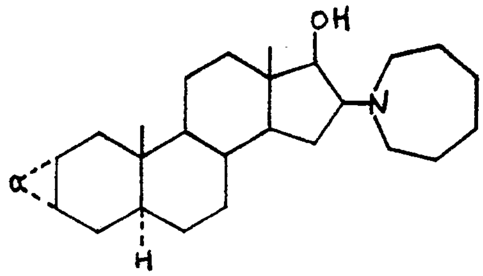

Formula 2

is reacted with a compound having the formula $NHR_2R_3$, wherein $R_2$ and $R_3$ have the same meaning as bove, that subsequently, if desired, the compound obtained is acylated and/or quaternised by methods obvious to those skilled in the art and that the non- and mono-quaternary ammonium compounds obtained are converted, if desired, into the acid addition salts thereof.

Compounds according to the present invention may be prepared by reacting a compound having the above formula 2 with the above compound having formula $NHR_2R_3$.

This reaction is carried out at an elevated temperature, usually between 70 and 250 °C, if necessary under pressure and in the presence of water. The compounds obtained by conducting this reaction are compounds according to the present invention with $R_1$ and $R_4$ both being H, which compounds may be acylated ($R_1$ and/or $R_4$ being an acyl group as discussed above).

The acylation reactions are carried out by reaction With an acylhalide at a temperature of 5-60 °C and preferably at room temperature in an organic solvent, like dichloromethane, for 1-48 hours, preferably for 1-30 hours. If acylation of the 17β-OH group is desired only, the amount of acylhalide to be used should be more or less equimolar. If the acylation of both the 17β-OH and the 3α-OH group is desired a molar excess of at least 3:1 of the acylhalide is required.

If only the 3α-OH group should be acylated the steroid is reacted with hydrogenchloride followed by an acylhalide in a molar ratio of about 1:1 (acylhalide:steroid). After this reaction step another acyl group may be incorporated at the 17β-OH group in order to prepare compounds according to the present invention having different acyl groups $R_1$ and $R_4$.

Another way to produce compounds having different acyl groups $R_1$ and $R_4$ is reacting the non-acylated steroid in equimolar amount with an acylhalide and subsequently with an acid anhydride, like succinic anhydride. Still another way to produce compounds having different acyl groups $R_1$ and $R_4$ is reacting 3α-0H,17β-acyl compounds with an acylhalide.

The quaternary ammonium compounds are obtained by allowing the non-quaternized compounds according to the present invention to react with an excess of an aliphatic hydrocarbon halide having 1-6 carbon atoms in a suitable solvent, such as methylene chloride, for one or several days at room temperature or for several hours or a few days at elevated temperature. The mono-quaternary compounds can be obtained by reacting the non-quaternized compounds according to the present invention with a restricted amount of hydrocarbon halide while reducing the reaction time, and separating the 2β-mono-quaternary and/or the 16β-mono-quaternary compound from the reaction mixture, e.g. by chromatography or by fractional crystallisation. Also use can be made of the fact that the 16β-mono-quaternary compound is sparingly soluble in certain solvents, e.g. ether. The reaction can then be performed in the presence of such solvent so that the 16β-mono-quaternary compound precipitates during the reaction or after the reaction the 16β-mono-quaternary compound is precipitated from the reaction mixture by the addition of such solvent. The 2β-mono-quaternary compound can be obtained from the mother liquor by e.g. chromatography on alumina.

The non-quaternary and mono-quaternary compounds can be converted into the acid addition salts thereof in the usual way by reaction with an pharmaceutically acceptable organic or inorganic acid e.g. in aqueous solution.

The present compounds are intended particularly for use in clinical practice to produce skeletal muscular paralysis during surgical operations.

The compounds are usually administered by intravenous injection, in initial dosages between 5 and 50 mg (bolus injection), followed if necessary by smaller supplementary dosages.

The present invention is further illustrated by way of the following examples.

## Example 1

A solution of (2α,3α,5α,16α,17β)-2,3,16,17-diepoxy-androstan-17-ol acetate (63 g) in hexamethyleneimine (350 ml) and water (30 ml) was heated under reflux for 30 min. The solvent was removed under reduced pressure and the residue was dissolved in methanol (500 ml). The stirred solution was cooled with an ice water bath and sodium borohydride (20 g) was added portionwise over 15 min. The solution was stirred for a further 40 min., then water (4 1) was added to precipitate the product as a gum. The gum was dissolved in dichloromethane and the solution was washed with water (2x), dried ($Na_2SO_4$) and evaporated to dryness. A small crop (17 g) was obtained from light petroleum but shown to be enriched in the minor 16α,17β-isomer. The crop and mother liquors were chromatographed separately on silica (0.063-0.2 mm), eluting with dichloromethane-ethanol-ammonia (200:10:1 v/v) to give (2α,3α,5α,16β,17β)-2,3-epoxy-16-(hexahydro-1H-azepin-1-yl)-androstan-17-ol (49 g). A sample crystallised from acetone had m.p. 117-125 °C; $[\alpha]_D^{20}$ = +20.1° (c = 0.92 in $CHCl_3$).

A solution of this compound (30 g) in piperidine (180 ml) and water (20 ml) was heated under reflux for 4 days. The solvent was removed under reduced pressure and the residue was dissolved in dichloromethane, washed with water (2x), dried ($Na_2SO_4$) and evaporated to dryness. Chromatography of the crude product on silica (650 g; 0.063-0.2 mm) eluting with dichloromethane-ethanol-ammonia (200:10:1 v/v) followed by crystallisation of the appropriate fractions from acetone, gave (2β,3α,5α,16α,17β)-16-(hexahydro-1H-azepin-1-yl)-2-(1-piperidinyl)-androstane-3,17-diol (12 g). A sample recrystallised from acetone had m.p. 150-164 °C; $[\alpha]_D^{20}$ = +85 ° (c = 0,97 in $CHCl_3$).

In a manner similar to that described above, the corresponding 2-(4-morpholinyl) compound was prepared and crystallised from dichloromethane-acetone, m.p. 164-176 °C; $[\alpha]_D^{20}$ = +68.5° (c = 1.35 in $CHCl_3$).

Likewise the corresponding compounds with a 1-pyrrodinyl [m.p. 183-187 °C; $[\alpha]_D^{20}$ = +31.0° (c = 1.28 in $CHCl_3$)], and a hexahydro-1H-azepin-1-yl [m.p. 133-145 °C; $[\alpha]_D^{20}$ = +76.2° (c = 0.93 in $CHCl_3$)] group at position 2 were prepared.

## Example 2

Acetyl chloride (2.5 ml) was added to a solution of (2β,3α,5α,16β,17β)-16-(hexahydro-1H-azepin-1-yl)-2-1-piperidinyl)-androstane-3,17-diol (1.3 g) in dichloromethane (20 ml). After 4 h. at room temperature, the solvent and the excess of acetyl chloride were removed under reduced pressure and the residue was dissolved in dichloromethane. The solution was washed with sodium carbonate solution (5% w/v) and with water (2x), dried ($Na_2SO_4$), and the solvent was removed under reduced pressure to give (2β,3α,5α,16β,17β)-16-(hexahydro-1H-azepin-1-yl)-2-(1-piperidinyl)-androstane-3,17-diol diacetate as a froth (1.35 g), $[\alpha]_D^{20}$ = +18.4° (c = 0.82 in $CHCl_3$).

In a similar way the corresponding compound having a hexahydro-1H-azepin-1-yl group at position 2 and the compounds (2β,3α,5α,16β,17β)-16-(hexahydro-1H-azepin-1-yl)-2-(4-morpholinyl)-androstane-3,17-diol diacetate, $[\alpha]_D^{20}$ = +16.4 ° (c = 1.35 in $CHCl_3$), and (2β,3α,5α,16β,17β)-16-(hexahydro-1H-azepin-1-yl)-2-(4-morpholinyl)-androstane-3,17-diol dipropanoate, as a colourless gum (1.5 g), were obtained.

## Example 3

Butanoyl chloride (2.0 ml) was added to a solution of (2β,3α,5α,16β,17β)-16-(hexahydro-1H-azepin-1-yl)-2-(1-piperidinyl)-androstane-3,17-diol (8 g), in dichloromethane (120 ml). After 18 h. at room temperature, the solution was evaporated to dryness under reduced pressure and the residue was dissolved in dichloromethane. The solution was washed with 5% sodium carbonate solution and with water (2x), dried ($Na_2SO_4$) and evaporated under reduced pressure. The crude product was chromatographed on silica (180 g; 0.063-0.2 mm), eluting with dichloromethane-ethanol-ammonia (200:10:1 v/v) to give (2β,3α,5α,16β,17β)-16-(hexahydro-1H-azepin-1-yl)-2-(1-piperidinyl)-androstane-3,17-diol 17-butanoate as a gum (6.6 g).

In a similar way the following 17-monoesters were prepared: 2-piperidino-17-propanoate, obtained as a gum $[\alpha]_D^{20}$ = +64.2° (c = 0.89 in $CHCl_3$)); 2-piperidino-17-(2,2-dimethylpropanoate), obtained as a gum (-$[\alpha]_D^{20}$ = +12.5. (c = 0.97 in $CHCl_3$)); 2-piperidino-17-cyclopentanecarboxylate, obtained as a gum ($[\alpha]_D^{20}$ = +59.4° (c= 0.95 in $CHCl_3$)); 2-homopiperidino-17-butanoate, obtained as a gum; 2-pyrrolidino-17-butanoate, obtained as a gum ($[\alpha]_D^{20}$ = +16.6° (c = 0.83 in $CHCl_3$)).

In a similar way the corresponding compound having a hexahydro-1H-azepin-1-yl group at position 2 and the compounds (2β,3α,5α,16β,17β)-16-(hexa-hydro-1H-azepin-1-yl)-2-(4-morpholinyl-androstane-3,17-diol 17-acetate, as a gum (1.8 g), and (2β,3α,5α,16β,17β)-16-(hexahydro-1H-azepin-1-yl)-2-(1-piperidinyl)-androstane-3,17-diol 17-(2-methylpropanoate), as a colourless gum (8.4 g), were obtained.

## Example 4

Acetyl chloride (1.15 ml) was added to a solution of (2β,3α,5α,16β,17β)-16-(hexahydro-1H-azepin-1-yl)-2-(4-morpholinyl)-androstane-3,17-diol hydrochloride (1:1) (4 g) in dichloromethane (200 ml). After 18 h. at room temperature, the solvent was removed under reduced pressure and the residue was redissolved in dichloromethane. The solution was washed with sodium carbonate solution (5% w/v) and with water (2x) and dried ($Na_2SO_4$). After removal of solvent under reduced pressure, the crude mixture was dissolved in dichloromethane and chromatographed on silica (150 g; 0.063-0.2 mm) eluting with dichloromethane-ethanol-ammonia (200:10:1 v/v). Removal of solvents from the appropriate fractions afforded (2β,3α,5α,16β,17β)-16-(hexahydro-1H-azepin-1-yl)-2-(4-morpholinyl)-androstane-3,17-diol 3-acetate as a gum (1.1 g).

## Example 5

Acetyl chloride (4 ml) was added to a solution of (2β,3α,5α,16β,17β)-16-(hexahydro-1H-azepin-1-yl)-2-(1-piperidinyl)-androstane-3,17-diol 17-butanoate (6.6 g) in dichloromethane (70 ml). After 18 h. at room temperature, the solvent and the excess of acetyl chloride were removed under reduced pressure and the intermediate hydrochloride salt of the product was precipitated from acetone-diethyl ether. The solid was dissolved in dichloromethane and the solution was washed with sodium carbonate solution (5% w/v) and with water (2x). The solution was dried ($Na_2SO_4$) and the solvent was removed under reduced pressure to give (2β,3α,5α,16β,17β)-16-(hexahydro-1H-azepin-1-yl)-2-(1-piperidinyl)-androstane-3,17-diol 3-acetate 17-butanoate as a yellow gum (5.1 g).

In a similar way the following 3,17-diesters were prepared: 2-piperidino-3-acetate-17-propanoate, obtained as a gum ($[\alpha]_D^{20}$ = +17.8° (c = 1.04 in $CHCl_3$)); 2-piperidino-3-acetate-17-(2,2-dimethylpropanoate), obtained as a gum ($[\alpha]_D^{20}$ +12.5° (c = 0.97 in $CHCl_3$)); 2-piperidino-3-acetate-17-cyclopentanecarboxylate, obtained as a gum ($[\alpha]_D^{20}$ = +15.8° (c = 0.87 in $CHCl_3$)); 2-homopiperidino-3-acetate-17-butanoate, obtained as a gum; 2-pyrrolidino-3-acetate-17-butanoate, obtained as a gum ($[\alpha]_D^{20}$ = +14.7° (c = 1.37 in $CHCl_3$)).

In a similar way the corresponding compound having a hexahydro-1H-azepin-1-yl group at position 2 was obtained.

In a similar manner, treatment of (2β,3α,5α,16β, 17β)-16- hexahydro-1H-azepin-1-yl)-2-(1-piperidinyl)-androstane-3,17-diol 17- (2-methylpropanoate) (8.4 g) with acetyl chloride (5.1 ml) gave the corresponding 3-acetate.

In a similar manner, treatment of (2β,3α,5α,16β, 17β)-16-(hexahydro-1H-azepin-1-yl)-2-(4-morpholinyl)-androstane-3,17-diol 3-acetate (1.1 g) with butanoyl chloride (2.2 ml) gave the corresponding 17-butanoate compound as a gum (0.75 g), after chromatography of the crude product on silica (30 g; 0.063-0.2 mm), as described previously.

## Example 6

A solution of (2β,3α,5α,16β,17β)-16-(hexahydro-1H-azepin-1-yl)-2-(1-piperidinyl)-androstane-3,17-diol diacetate (4.4 g) (described in Example 2) was dissolved in methanol (22 ml) and the solution was allowed

to stand at room temperature for 16 h. The solvent was removed and the resiudue (3.8 g) was crystallised from acetone to give a solid, which was mainly the diol. The material from the mother liquor was chromatographed on a column of silica (90 g). Elution with dichloromethane-methanol-ammonia (300:10:1 v/v) gave (2β,3α,5α,16β,17β)-16-(hexahydro-1H-azepin-1-yl)-2-(1-piperidinyl)-androstane-3,17-diol 3-acetate as a gum (1.4 g), $[\alpha]_D^{20}$ = +30.0° (c = 0.68 in $CHCl_3$).

## Example 7

Bromomethane (20 g) was added to a solution of (2β,3α,5α,16β,17β)-16-(hexahydro-1H-azepin-1-yl)-2-(1-piperidinyl)-androstane-3,17-diol 3-acetate 17-butanoate (24 g) in dichloromethane (300 ml) and the solution was set aside at room temperature for 24 h. The solvent was removed under reduced pressure and the crude product was precipitated from dichloromethane solution by the addition of diethyl ether. The crude product (24.8 g) was chromatographed on alumina (750 g; Fluka basic type 5016A). The appropriate fractions were combined and evaporated to dryness to give the product, which was dissolved in acetone and reprecipitated by the addition of diethyl ether. Recrystallisation from dichloro-ethane-acetone-diethyl ether gave 1-[(2β,3α,5α,16β,17β)-3-(acetyloxy)-17-(1-oxobutoxy)-2-(1-piperidinyl)-androstan-16-yl]-hexahydro-1-methyl-1H-azepinium bromide (19.8 g), m.p. 202-207 °C (decomp.); $[\alpha]_D^{20}$ = -10.4° (c = 0.77 in $CHCl_3$).

In a similar way the following compounds were obtained:

1. 1-[(2β,3α,5α,16β,17β)-3,17-bis(acetyloxy)-2-(1-piperidinyl)-androstan-16-yl]hexahydro-1-methyl-1H-azepinium bromide, m.p. 195-208 °C (decomp.); $[\alpha]_D^{20}$ = -12.9° (c = 1.0 in $CHCl_3$);

2. 1-[(2β,3α,5α,16β,17β)-3-(acetyloxy)-17-(2-methyl-1-oxopropopxy)-2-(1-piperidinyl)-androstan-16-yl]-hexahydro-1-methyl-1H-azepinium bromide, a non- crystalline solid, m.p. 190-197 °C (decomp.) ; $[\alpha]_D^{20}$ = -9,6° (c = 1.26 in $CHCl_3$);

3. 1-[(2β,3α,5α,16β,17β)-3-hydroxy-17-(1-oxobutoxy)-2-(1-piperidinyl)-androstan-16-yl]hexahydro-1-methyl-1H-azepinium bromide, a non-crystalline solid, m.p. 169-175 °C (decomp.); $[\alpha]_D^{20}$ = +23.9° (c = 1.01 in $CHCl_3$);

4. 1-[(2β,3α,5α,16β,17β)-3,17-bis(acetyloxy)-2-(4-morpholinyl)-androstan-16-yl]hexahydro-1-methyl-1H-azepinium bromide, a non-crystalline solid, m.p. 190-195 °C (decomp.) ;$[\alpha]_D^{20}$ = -14.4° (c = 1.14 in $CHCl_3$);

5. 1-[(2β,3α,5α,16β,17β)-3,17-bis(1-oxopropoxy)2-(4-morpholinyl)-androstan-16-yl]hexahydro-1-methyl-1H-azepinium bromide, a non-crystalline solid, m.p. 197-205 °C (decomp.); $[\alpha]_D^{20}$ = -13.6° (c = 0.95 in $CHCl_3$);

6. 1-[(2β,3α,5α,16β,17β)-17-(acetyloxy)-3-hydroxy-2-(4-morpholinyl)-androstan-16-yl]hexahydro-1-methyl-1H-azepinium bromide, a non-crystalline solid, m.p. 181-186 °C (decomp.); $[\alpha]_D^{20}$ = +14.1° (c = 1.12 in $CHCl_3$);

7. 1-[(2β,3α,5α,16β,17β)-3-(acetyloxy)-2-(4-morpholinyl-17-(1-oxobutoxy)-androstan-16-yl]hexahydro-1-methyl-1H-azepinium bromide, m.p. 217-222 °C (decomp.), $[\alpha]_D^{20}$ = -13.6° (c = 1.13 in $CHCl_3$);

8. 1-[(2β,3α,5α,16β,17β)-3,17-bis(acetyloxy)-2-(4-morpholinyl)-androstan-16-yl]hexahydro-1-(2-propenyl)-1H-azepinium bromide as an off-white solid (4.8 g), m.p. 183-192 °C (decomp.); $[\alpha]_D^{20}$ = -16.1° (c = 1.22 in $CHCl_3$);

9. 1-[(2β,3α,5α,16β,17β)-3,17-bis(1-oxopropoxy)-2-(4-morpholinyl)-androstan-16-yl]hexahydro-1-(2-propenyl)-1H-azepinium bromide, m.p. 164-172 °C (decomp.); $[\alpha]_D^{20}$ = -15.6° (c = 1.01 in $CHCl_3$);

10 1-[(2β,3α,5α,16β,17β)-3-(acetyloxy)-17-(1-oxobutoxy-2-(1-piperidinyl)-androstan-16-yl]hexahydro-1-(2-propenyl)-1H-azepinium bromide, m.p. 173-178 °C (decomp.); $[\alpha]_D^{20}$ = -11.6° (c = 1.13 in $CHCl_3$);

11 1-[(2β,3α,5α,16β,17β)-17-(acetyloxy)-3-hydroxy-2-(4-morpholinyl)-androstan-16-yl]hexahydro-1-(2-propenyl)-1H-azepinium bromide, a non-crystalline solid, m.p. 151-155 °C; $[\alpha]_D^{20}$ = +12.6° (c = 0.95 in $CHCl_3$);

12 1-[(2β,3α,5α,16β,17β)-3-(acetyloxy)-17-(1-oxobutoxy)-2-(1-piperidinyl)-androstan-16-yl]hexahydro-1-(2-propynyl)-1H-azepinium bromide, as a non-crystalline solid (1.1 g), m.p. 180-184 °C (decomp.); $[\alpha]_D^{20}$ = -10.8° (c = 1.14 in $CHCl_3$);

13 1-[(2β,3α,5α,16β,17β)-3-(acetyloxy)-17-(1-oxobutoxy)-2-(1-piperidinyl)-androstan-16-yl]hexahydro-1-ethyl-1H-azepinium bromide, a non-crystalline solid (1.2 g), m.p. 182-186 °C (decomp.); $[\alpha]_D^{20}$ = -12.4° (c = 1.10 in $CHCl_3$);

14 1-[(2β,3α,5α,16β,17β)-3,17-bis(acetyloxy)-2-(hexa-hydro-1H-azepin-1-yl)-androstan-16-yl]-hexahydro-1-methyl-1H-azepinium bromide; m.p. 190-204 °C (decomp.); $[\alpha]_D^{20}$ = +5.7° (c = 0.97 in $CHCl_3$);

15 1-[(2β,3α,5α,16β,17β)-3-(acetyloxy)-17-(1-oxobutoxy)-2-(hexahydro-1H-azepin-1-yl)-androstan-16-yl]hexahydro-1-methyl-1H-azepinium bromide; and m.p. 195-211 °C (decomp.); $[\alpha]_D^{20}$ = -1.5° (c = 0.86 in $CHCl_3$);

16 1-[(2β,3α,5α,16β,17β)-3-(acetyloxy)-17-(1-oxobutoxy)-2-(hexahydro-1H-azepin-1-yl)-androstan-16-yl]hexahydro-1-(2-propenyl)-1H-azepinium bromide m.p. 163-175 °C (decomp.); $[\alpha]_D^{20}$ = -3.7° (c = 0.83 in $CHCl_3$);

17 1-[(2β,3α,5α,16β,17β)-3-(acetyloxy)-17-(1-oxopropoxy)-2-(1-piperidinyl)-androstan-16-yl]hexahydro-1-methyl-1H-azepinium bromide, m.p. 193-197 °C (decomp.); $[\alpha]_D^{20}$ = -9.0° (c = 0.59 in $CHCl_3$);

18 1-[(2β,3α,5α,16β,17β)-3-hydroxy-17-(1-oxopropoxy)-2-(1-piperidinyl)-androstan-16-yl]hexahydro-1-methyl-1H-azepinium bromide, m.p. 178-185 °C (decomp.); $[\alpha]_D^{20}$ = +23.7° (c = 0.92 in $CHCl_3$);

19 1-[(2β,3α,5α,16β,17β)-3-(acetyloxy)-17-(2,2 dimethyl-1-oxopropoxy)-2-(1-piperidinyl)-androstan-16-yl]hexahydro-1-methyl-1H-azepinium bromide, m.p. 182-187 °C (decomp.); $[\alpha]_D^{20}$ = -6.5° (c = 0.9 in $CHCl_3$); 20 1-[(2β,3α,5α,16β,17β)-3-(acetyloxy)-17-[(cyclopentylcarbonyl)oxy]-2-(1-piperidinyl)-androstan-16-yl]hexahydro-1-methyl-1H-azepinium bromide, m.p. 206-211 °C (decomp.); $[\alpha]_D^{20}$ = -9.1° (c = 0.77 in $CHCl_3$);

21 1-[(2β,3α,5α,16β,17β)-3,17-dihydroxy-2-(1-piperidinyl)-androstan-16-yl]hexahydro-1-methyl-1H-azepinium bromide, m.p. decomp. above 215 °C; $[\alpha]_D^{20}$ = +45.1. (c = 1.18 in $CHCl_3$);

22 1-[(2β,3α,5α,16β,17β)-3-(acetyloxy)-17-hydroxy-2-(1-piperidinyl)-androstan-16-yl]hexahydro-1-methyl-1H-azepinium bromide, m.p. 182-186 °C ; $[\alpha]_D^{20}$ = +8.4° (c = 0.68 in $CHCl_3$);

23 1-[(2β,3α,5α,16β,17β)-3-(acetyloxy)-17-(1-oxobutoxy)-2-(1-pyrrolidinyl)-androstan-16-yl]hexahydro-1-methyl-1H-azepinium bromide, m.p. 191-195 °C; $[\alpha]_D^{20}$ = -15.6. (c = 1.23 in $CHCl_3$);

24 1-[(2β,3α,5α,16β,17β)-3-(acetyloxy)-17-(1-oxobutoxy]-2-(1-pyrrolidinyl)-androstan-16-yl]hexahydro-1-(2-propenyl)-1H-azepinium bromide, m.p. 166-170 °C; $[\alpha]_D^{20}$ -16.1° (c = 0.88 in $CHCl_3$).

## Claims

1. Compounds having the formula:

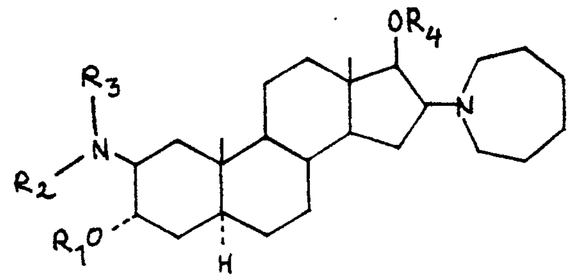

wherein $R_1$ is H or an acyl group having 1-12 carbon atoms,
$R_2$ and $R_3$ are an alkyl or aralkyl group having 1-12 carbon atoms, or When taken together form with the nitrogen atom a heterocyclic amino radical; and $R_4$ is H or an acyl group having 1-12 carbon atoms; and mono- or bisquaternary ammonium compounds thereof and acid addition salts of the non- or mono-quaternary ammonium compounds.

2. Compounds according to claim 1, characterized in that $R_1$ is H or an acyl group having 1-6 carbon atoms.

3. Compounds according to claims 1 or 2, characterized in that $R_2$ and $R_3$, when taken together, form with the nitrogen atom a heterocyclic amino radical.

4. Compounds according to claims 1-3, characterized in that $R_4$ is H or an acyl group having 1-6 carbon atoms.

5. Compounds according to claims 1-4, characterized in that these compounds are 16β-monoquaternary ammonium compounds.

6. Process for the preparation of the compounds according to claim 1, characterized in that a compound having the formula:

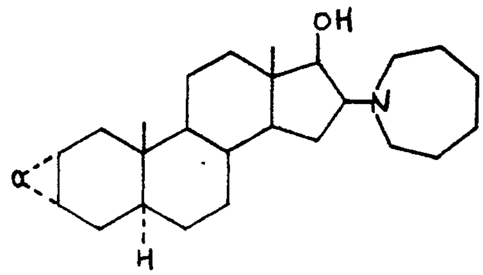

is reacted with a compound having the formula $NHR_2R_3$, wherein $R_2$ and $R_3$ have the same meaning as in claim 1, that subsequently, if desired, the compound obtained is acylated and/or quaternised by methods obvious to those skilled in the art and that the non- and monoquaternary ammonium compounds obtained, if desired, are converted into the acid addition salts thereof.

European Patent Office

# EUROPEAN SEARCH REPORT

Application Number

EP 89 20 0298

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | GB-A-1 138 605 (ORGANON LABORATORIES LTD)<br>* Claims *<br>--- | 1-6 | C 07 J 43/00<br>A 61 K 31/58 |
| A | JOURNAL OF MEDICINAL CHEMISTRY, vol. 16, no. 10, October 1973, pages 1116-1124, Washington, D.C., US; W.R. BUCKETT et al.: "Pancuronium bromide and other steroidal neuromuscular blocking agents containing acetylcholine fragments"<br>* Whole document *<br>--- | 1-6 | |
| A | EP-A-0 067 480 (AKZO N.V.)<br>* Claims *<br>----- | 1-6 | |

TECHNICAL FIELDS SEARCHED (Int. Cl.4)

C 07 J 43/00

The present search report has been drawn up for all claims

| Place of search | Date of completion of the search | Examiner |
|---|---|---|
| THE HAGUE | 11-04-1989 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document